# EUROPÄISCHE PATENTSCHRIFT

(11) **EP 1 942 961 B1**
(45) Veröffentlichungstag und Bekanntmachung des Hinweises auf die Patenterteilung: **09.10.2013**
(21) Anmeldenummer: 06806240.5
(22) Anmeldetag: 12.10.2006
(51) Int. Cl.: A61L 27/30, A61L 27/56

(54) **Verfahren zur Herstellung einer offenporigen biokompatiblen Oberflächenschicht für ein Implantat**
Method for the production of an open-cell biocompatible coating for an implant
Procédé de fabrication d'une couche de surface biocompatible à pores ouverts destinée à un implant.

(30) Priorität: 02.11.2005 DE 102005052354
(43) Veröffentlichungstag der Anmeldung: 16.07.2008
(73) Patentinhaber: Smith & Nephew Orthopaedics AG, 6343 Rotkreuz (CH)
(72) Erfinder: LERF, Reto, CH-4513 Langendorf (CH); SCHMOTZER, Hans, CH-8903 Birmensdorf (CH); SIEGMANN, Stephan, CH-3604 Thun (CH)
(74) Vertreter: Popp, Eugen
(86) Internationale Anmeldenummer: PCT/EP2006/009886
(87) Internationale Veröffentlichungsnummer: WO 2007/051519

(56) Entgegenhaltungen:
- EP-A- 1 035 230
- WO-A-01/19556
- WO-A-01/72664
- WO-A-86/06617
- WO-A-2006/020090
- DE-A1- 10 022 162

## Beschreibung

Die Erfindung betrifft Verfahren zur Herstellung einer offenporigen biokompatiblen Oberflächenschicht gemäß den Oberbegriffen der Patentansprüche 1 und 2.

Implantate und insbesondere Gelenkersatzimplantate gewinnen immer mehr an Bedeutung in der restaurativen und kurativen Medizin. Bei zementfreien Gelenkersatzimplantaten steht hierbei die mechanisch stabile Verankerung des Implantats im Knochen im Vordergrund. Diese ist wesentlich für die langfristige Haltbarkeit und Verträglichkeit der Implantate. Hierbei steht im Vordergrund, daß dem Knochen eine optimierte Oberfläche des Gelenkersatzimplantats geboten wird, so daß dieser sich optimal mit dem Implantat verbinden kann. Somit ist die Oberflächenstruktur bzw. -beschichtung eines Gelenkersatzimplantats von entscheidender Bedeutung, da durch diese eine Verbindung des Knochens mit dem Implantat, respektive der Implantatoberfläche ermöglicht oder, im ungünstigen Fall, ver- bzw. behindert wird.

Zur Herstellung solcher, ein Anwachsen fördernder Oberflächenschichten haben sich bislang poröse Schichten bewährt. So sind verschiedene Verfahren bekannt, mit denen sich poröse Schichten herstellen lassen. Als Materialien werden hierbei biokompatible Materialien, insbesondere Metalle, wie beispielsweise Titan eingesetzt. Die Oberflächenschichten werden zum überwiegenden Teil auf Knochenimplantaten angeordnet, so daß deren Langzeitverankerung im Knochen verbessert ist. Die notwendigen porösen Schichten können beispielsweise mittels Sintertechnik erzeugt werden, wobei die Strukturen und die Sinterbedingungen so gewählt werden, daß Hohlräume zwischen den auf die Oberfläche aufgebrachten Metall- bzw. Titanteilchen erhalten bleiben.

Hinsichtlich der zementfreien Verankerung von Gelenkersatzimplantaten können zwei Arten unterschieden werden. Es handelt sich hierbei zum einen um ein Knochenanwachsen sowie zum anderen um ein Knocheneinwachsen.

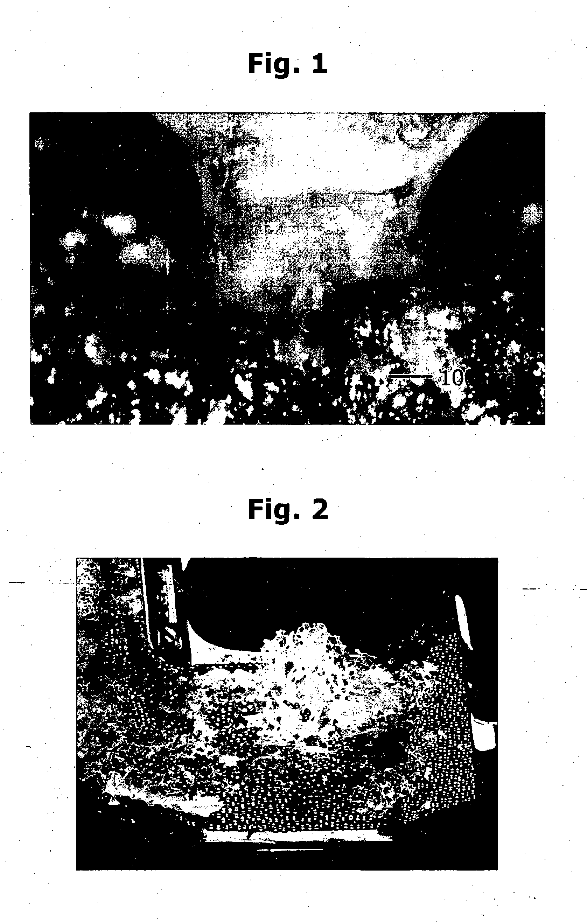

Beim Knochenanwachsen haften Knochenzellen auf der Oberfläche. Damit eine genügende Festigkeit der Verbindung Knochen-Implantat entsteht, muß die Oberfläche eine gewisse Rauheit aufweisen. Beispiele dafür sind mittels Korundstrahlen aufgerauhte Implantate oder Vakuum-Plasma-Titan-Spritzschichten, wie sie beispielsweise in Fig. 1 als Stand der Technik dargestellt sind. Fig. 1 zeigt ein auf einer gestrahlten Oberfläche einer Prothese aus einer Ti6A17Nb-Legierung angewachsenes Knochenbälkchen.

Hinsichtlich eines Knochenanwachsens zur Verbindung eines Knochens mit einem Implantat ist zu beachten, daß eine Lastübertragung im Sinne einer Zugbelastung nur in sehr beschränktem Umfang möglich ist, da bei einer Zugbeanspruchung die Knochenzellen einfach von der Oberfläche abgehoben werden.

Bei einem Knocheneinwachsen wächst Knochen in auf bzw. an der Oberfläche des Implantats vorhandene Hohlräume ein. Beispiele für solche Oberflächen sind offenporige Kugel- oder Drahtoberflächen, die beispielsweise mittels Sintertechnik hergestellt werden können. Weit verbreitet sind im Hinblick auf die erstgenannten Kugeloberflächen die sogenannten "Beads"-Beschichtungen. Eine solche Beschichtung ist in Fig. 2 gezeigt, die eine "Beads"-Beschichtung aus einer CoCr-Legierung mit einem darauf eingewachsenen Knochen als Makroaufnahme zeigt.

Ein stabiles Anwachsen des Knochens auf einer solchen Implantatoberfläche ist jedoch vielfach dennoch nicht gegeben. Darüber hinaus benötigt ein Hineinwachsen in eine solche Kugel- oder Drahtoberfläche deutlich mehr Zeit als ein Anwachsen des Knochens, wobei durch eine auf einer Kugel- oder Drahtoberfläche entstehende Verzahnung des Knochens mit der Oberfläche in gewissem Maß auch Zugkräfte übertragen werden können.

Aus der Literatur sind verschiedene Verfahren bekannt, mit welchen Oberflächen zum Knochenanwachsen hergestellt werden können.

So beschreibt V. Galante et a., JBJS, 53A (1971), S. 101-114 beispielsweise, daß ein Geflecht aus feinen Titandrähten auf ein Substrat gesintert wird.

Die beiden US-Patente 3,855,638 und 5,263,986 offenbaren ein Verfahren, bei dem ein Titanpulver aus kugeligen Partikeln verschiedener Größe auf ein Substrat aufgesintert wird.

Das US-Patent 4,206,516 verwendet hingegen ein gemahlenes Titanhydridpulver aus kantigen Partikeln, das wiederum auf ein Substrat aufgesintert wird.

Mit diesen Verfahren ist es möglich, offenporige Schichten herzustellen, in deren Poren Knochen einwachsen können. Diese mittels Sintertechnik hergestellten porösen Strukturen können durch eine Steuerung der Sinterbedingungen erzeugt werden, wobei es möglich ist, Hohlräume zwischen den auf die Oberfläche des Substrats aufgebrachten Titanteilchen zu erhalten.

Ferner ist es möglich, aus einem Gemisch aus thermisch instabilen Platzhaltern und Titanpulver oder aus Platzhaltern und Titanhydridpulver mittels Sintern ein Skelett aus Titan herzustellen. Diesbezügliche Verfahren sind beispielsweise in den Patentschriften US 5,034,186 oder WO 01/19556 beschrieben; ebenso widmet sich ein Verfahren der Firma Intermedics, Austin, USA, unter dem Titel "Cancellous Structured Titanium" einer solchen Möglichkeit.

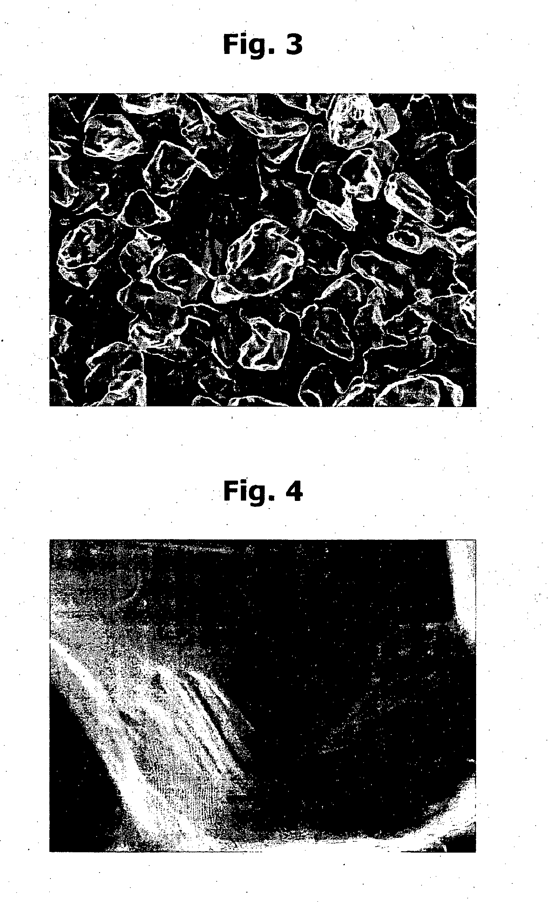

Allen über einen solchen Sinterprozeß hergestellten Titanschichten ist es jedoch inhärent nachteilig, daß die Rauheit der ehemaligen Titanpartikel oder Titanfasern durch eine Oberflächendiffusion eingeglättet wird. Der Knochen kann zwar in die Poren einwachsen, aber mikroskopisch gesehen, finden die Knochenzellen kaum Halt auf der glatten Titan-oberfläche. Dieser Sachverhalt ist in den Figuren 3 und 4 dargestellt, wobei Fig. 3 eine rasterelektronenmikroskopische Aufnahme einer nach dem vorgenannten US-Patent 4,206,516 hergestellten Titan-Beschichtung in einer 50-fachen Vergrößerung zeigt. Fig. 4 zeigt ebenfalls eine rasterelektronenmikroskopische Aufnahme derselben Titan-Beschichtung, jedoch in einer 1000-fachen Vergrößerung. Die durch die Oberflächendiffusion auf mikroskopischer Ebene eingeglättete Oberfläche ist gut zu erkennen.

Dieser Nachteil kann umgangen werden, indem die Zeit, in dem die Titanteilchen hohen Temperaturen ausgesetzt sind, drastisch reduziert wird.

Aus der Literatur sind hierzu verschiedene Verfahren bekannt, mit welchen Oberflächen zum Knochenanwachsen dargestellt werden können. Wie bereits erwähnt, bilden thermische Spritzverfahren, namentlich das Vakuum-Plasma-Spritzen, eine Möglichkeit, eine rauhe Titan-Oberfläche industriell herzustellen. Die Verfahrensparameter können beim thermischen Spritzen so gewählt werden, daß die Partikel eines Schichtwerkstoffs nur kurzzeitig erhitzt werden, wobei sie höchstens teilweise aufschmelzen und beim Auftreffen auf das Substrat rasch abgeschreckt werden. Durch diese Verfahrensführung bleibt die rauhe Oberfläche des Spritzpulvers teilweise erhalten und resultiert in einer sichtbaren topographischen Rauheit der gespritzten Schicht. Diesbezügliche Lösungen zur Erzeugung einer rauhen Oberfläche sind beispielsweise in dem US-Patent 4,542,539, DE 10022162 A oder in den Artikeln AESCULAP, Wissenschaftliche Information 22: "Die PLASMAPORE-Beschichtung für die zementlose Verankerung von Gelenkendoprothesen" sowie in "Osteointegration, Oberflächen und Beschichtungen orthopädischer Implantate für den zementfreien Einsatz" der PI Precision Implants AG beschrieben.

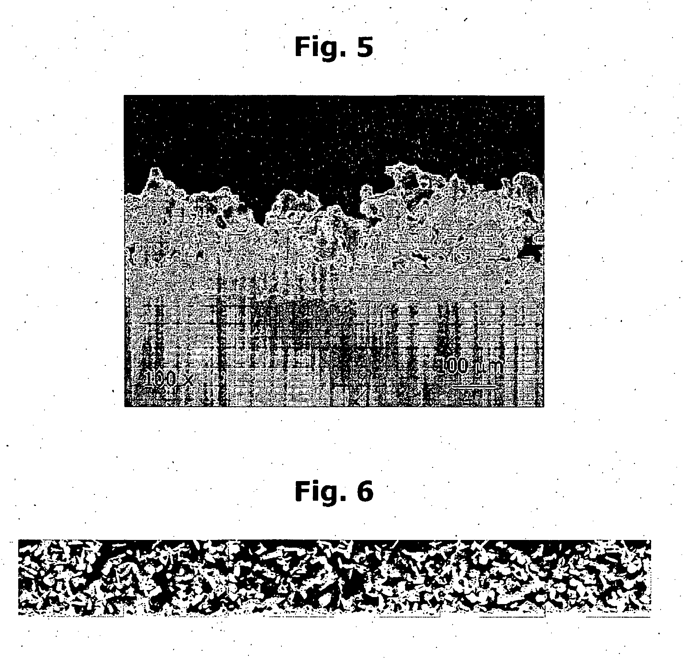

Dem Gewinn an Rauhigkeit, der mit dem Vakuum-Plasma-Spritzverfahren erzielt werden kann, steht jedoch ein gravierender Nachteil gegenüber, da Flamm- oder Plasma-gespritzte Titanschichten praktisch keine nach außen offenen Poren aufweisen und somit ein Einwachsen des Knochens per se verhindern. Diese Nachteile konnten auch durch die in vorgenannten Druckschriften genannten Verfahren nicht gelöst werden. Ein Beispiel für eine mittels Vakuum-Plasma-Spritzen aufgebrachte Titanschicht ist in Fig. 5 dargestellt. Diese Figur zeigt einen metallographischen Schliff durch eine Vakuum-Plasma-gespritzte Titanschicht als rasterelektronenmikroskopische Aufnahme in 100-facher Vergrößerung. Gut zu erkennen ist die Rauheit der auf das Substrat aufgebrachten Titanschicht.

Ferner sind andere Verfahren bekannt, die auf chemischem oder elektrochemischem Weg eine rauhe Oberfläche erzeugen, auf der Knochen besser anwachsen kann. Es handelt sich hierbei um Ätz- oder Anodisierverfahren, die beispielsweise in P.-I. Branemark et al., DE 300 74 46 C3 oder S. Steinemann et al., US 5,456,723 beschrieben sind.

Während die bisher präsentierten Techniken entweder auf Knochenanwachsen oder Knocheneinwachsen ausgelegt waren, gibt es neuere Entwicklungen, die eine Kombination beider zementfreier Verankerungsarten im Knochen ermöglichen. Eine solche Oberfläche ist beispielsweise unter den Handelsnamen Hedrocel^{®} oder "Trabecular Metal" bekannt. Hedrocel^{®} ist eine Oberfläche, mittels der eine Verankerung des Knochens möglich ist. Die Herstellung dieser Oberfläche ist jedoch sehr aufwendig und teuer. Hierbei wird, ausgehend von einem hochporösem Schaum aus Kohleglas, eine offenporige Tantalstruktur erzielt, indem in einem CVD-Prozeß Tantal auf ein Kohleglasgerüst abgeschieden wird. Zum Anbringen dieser porösen Struktur auf einer Oberfläche eines Knochenimplantats ist ein weiterer CVD-Prozeß notwendig, wobei die Prozeßtemperatur mehr als 900°C beträgt, was zu einer Schädigung des Gefüges des Substrats, also des lasttragenden Implantats, führen kann. Der zweistufige Herstellungsprozeß der Hedrocel^{®}-Oberfläche ist in den Druckschriften R.B. Kaplan, US 5,282,861 und R.C. Cohen et al., US 6,063,442 beschrieben.

Ein weiteres Verfahren, das in der europäischen Patentanmeldung EP 1 449 544 A1 beschrieben ist, zielt darauf ab, die Oberfläche von gesinterten Partikeln nachträglich aufzurauhen. Dieser Aufrauhvorgang wird mittels eines naßchemischen Ätzprozesses durchgeführt, so daß die Herstellung der aufgerauhten Oberfläche wiederum in einem zweistufigen Verfahren resultiert, nämlich in einer vorherigen Aufbringung von gesinterten Partikeln auf einem Substrat und einem anschließenden naßchemischen Ätzen. Dieses Verfahren birgt zudem die Gefahr, daß unerwünschte und oft sogar gefährliche Rückstände aus dem Ätzprozeß in den Poren der aufgebrachten Oberflächenschicht verbleiben.

Andere Technologien haben zum Ziel, poröse Schichten, wie sie zum Beispiel sintertechnisch hergestellt werden, zusätzlich mit einer sehr dünnen, das Knochenanwachsen fördernden Substanz zu versehen. Häufig ist diese Substanz Hydroxyapatit, also der mineralische Bestandteil des Knochens. Ein solches Verfahren ist beispielsweise in der US 5,279,831 beschrieben, wobei dort explizit die Aufbringung auf bereits porös eingestellte Oberflächen beansprucht wird. Die US 6,426,114 beschreibt die Aufbringung einer solchen Beschichtung mittels eines Sol-Gel-Prozesses. In aller Regel sind die so erzielten Hydoxyapatit-Schichten weniger als 1 µm dick und können daher auf eine beliebige Oberfläche, also auch auf eine poröse Oberfläche, appliziert werden.

Gemäß dem Vorgenannten läßt sich zusammenfassend festhalten, daß es bisher vielfältige Versuche gab, eine für ein Knocheneinwachsen zufriedenstellend strukturierte Oberfläche auf einem Gelenkersatzimplantat herzustellen. Bislang ist es jedoch lediglich gelungen,
- offenporöse Beschichtungen herzustellen, in die der Knochen einwachsen kann, die im Submikrometerbereich jedoch keine topographischen Anreize für eine Osteoplasten-Adhäsion und damit für ein schnelles bzw. gegenüber dem Stand der Technik besseres Knochenanwachsen geben;
- ein Oberflächenverfahren zur Verfügung zu stellen, das eine Rauheit von einigen Mikrometern und eine Submikrometerstruktur für eine bessere Adhäsion der Osteoplasten bietet, wobei diese Oberflächen jedoch keine offene Porosität aufweisen, in die der Knochen einwachsen könnte;
- Beschichtungen mit einer großen Rauheit im Bereich von einigen 10 Mikrometern herzustellen, die eine beschränkte Porosität aufweisen, aber wiederum keine eigentliche geeignete, d.h. Submikrometerstruktur besitzen;
- offenporöse Beschichtungen herzustellen, in die der Knochen einwachsen kann und deren Oberfläche eine Submikrometer-Rauhigkeit aufweist, die jedoch keine ausreichend hohe Makro-Rauhigkeit aufweisen und sich somit nicht im Knochen "verkrallen" können.

Somit ist es das Ziel der vorliegenden Erfindung, die vorgenannte Lücke zu schließen, wobei es die Aufgabe der Erfindung ist, die Oberfläche eines Gelenkersatzimplantats so zu gestalten, daß die Oberfläche stabile nach außen offene Hohlräume aufweist, die eine Größe haben, die ausreichend ist, daß vaskularisiertes Knochengewebe hineinwachsen kann, wobei die Oberfläche eine sehr gute Biokompatibilität im Sinne einer bioinerten oder leicht bioaktiven Eigenschaft sowie eine gezielt einstellbare Submikrometer-Dimensionierung aufweist, die den Osteoplasten als Verankerungspunkte dienen kann, wobei die Herstellung der Oberfläche in einem einfachen und einstufigen Verfahren möglich ist und ein zu beschichtendes Substrat thermisch nur gering belastet wird.

Diese Aufgabe wird durch Verfahren zur Herstellung einer offenporigen biokompatiblen Oberflächenschicht gemäß den Patentansprüchen 1 und 2 gelöst.

Eine beispielhafte offenporige biokompatible Oberflächenschicht für ein Implantat, die durch die hier beanspruchten Verfahren geschaffen wird, ist über einer Rohoberfläche des Implantats angeordnet, wobei Poren der offenporigen Oberflächenschicht zu einem zusammenhängenden Porennetzwerk verbunden sind und die Oberflächenschicht eine innere Oberfläche von ≥ 0,06 µm/µm², vorzugsweise von ≥ 0,035 µm/µm² und besonders bevorzugt von ≥ 0,025 µm/µm², bildanalytisch gemessen als 2D-Berandungslinie pro Flächeneinheit im metallographischen Schliff bei 100-facher Vergrößerung, aufweist.

Ein wesentlicher Punkt der Erfindung liegt darin, daß die offenporige biokompatible Oberflächenschicht durch die erfindungsgemäßen Verfahren ein Porennetzwerk aufweist, dessen Poren auch innerhalb der Schicht zusammenhängend ausgebildet sind, so daß in die Oberflächenschicht einwachsendes Knochengewebe das Porennetzwerk vollständig durchdringen kann und sich auf diese Weise eine das Porennetzwerk durchziehende Knochenstruktur aufbaut, die unter anderem auch innerhalb der Oberflächenschicht in das Porennetzwerk bildenden Kanälen der Oberflächenschicht verläuft. Auf diese Weise durch- und unterwandert das einwachsende Knochengewebe die Oberfläche der Oberflächenschicht, respektive das zusammenhängende Porennetzwerk auf eine Weise, die eine innige Verbindung der Oberflächenschicht mit dem Knochengewebe gewährleistet und ein Ablösen des Knochengewebes von der Oberflächenschicht, beispielsweise unter Zugbelastung, verhindert.

Ein weiterer wesentlicher Punkt der Erfindung besteht darin, dass durch die erfindungsgemäßen Verfahren eine innere Strukturierung der Oberflächenschicht mit einer großen inneren Oberfläche geschaffen wird, die eine große Rauheit aufweist, so daß ein Anwachsen von Knochengewebe an diese Oberfläche zusätzlich begünstigt ist. Es sei an dieser Stelle erwähnt, daß selbstverständlich auch die äußere Oberfläche der Oberflächenschicht eine solche Rauheit aufweist.

Insbesondere die Kombination einer Oberflächenschicht mit einer großen inneren Oberfläche zusammen mit dem zusammenhängenden Porennetzwerk, das innerhalb der offenporigen Oberflächenschicht ausgebildet ist, gewährleistet eine optimierte Verbindung eines Knochens mit der Oberflächenschicht, dessen Gewebe in die Oberflächenschicht einwächst und unter anderem in hohem Maße auch in unterhalb der Oberfläche der Oberflächenschicht verlaufenden Kanälen des Porennetzwerkes verläuft.

Somit weist die Oberflächenschicht stabile, gegen außen offene Hohlräume auf, die eine Größe haben, daß vaskularisiertes Knochengewebe hineinwachsen kann und deren durchschnittlicher Durchmesser im Bereich von etwa 200 µm liegt.

Gemäß einem Beispiel weist die Oberflächenschicht in einem Abstand von 300 µm von der Rohoberfläche des Implantats entfernt eine Scherkraft von ≥ 350 N, bevorzugt von ≥ 500 N und besonders bevorzugt von ≥ 1000 N auf (gemessen gemäß S. Siegmann, M. Dvorak, H. Grützner, K. Nassenstein und A. Walter: Shear testing for characterizing the adhesive and cohesive coating strength without the need of adhesives; Proceedings of ITSC 2005 Thermal Spray connects: Explore its surfacing potential! (2005), p. 823-829, ISBN 3-87155-793-5). Ferner beträgt eine erforderliche Schnittarbeit zum Abscheren der Schicht in einem Abstand von 300 µm von der Rohoberfläche des Implantats entfernt ≥ 0,01 J, bevorzugt ≥ 0,05 J und besonders bevorzugt ≥ 0,1 J.

Des weiteren beträgt ein relativer Abrieb der Oberflächenschicht bei einer Porosität von 40 % - 50 % und einer Schichtdicke von 0,7 mm - 1,0 mm, gemessen in Anlehnung an den Miller-Test nach ASTM G75-01 ohne Abrasionsmedium, ≤ 0,1 mg/min, bevorzugt ≤ 0,05 mg/min und besonders bevorzugt ≤ 0,01 mg/min.

Des weiteren weist die Oberflächenschicht eine Verbindung zwischen den die Oberfläche bildenden Partikeln sowohl untereinander als auch eine Verbindung der Partikel mit dem Substrat, d. h. mit der Rohoberfläche des Implantats auf, die als Sinterhals bezeichnet wird. Der durchschnittliche Durchmesser der Sinterhälse der erfindungsgemäßen Oberflächenschicht beträgt ≥ 5 µm, bevorzugt ≥ 15 µm und besonders bevorzugt ≥ 25 µm. Ein solches Schliffbild ist beispielsweise in Fig. 6 dargestellt. Dort lassen sich sowohl die Art als auch die Dimension dieser Sinterhälse gut erkennen. Die vorgenannten Dimensionsangaben der Sinterhälse wurden durch Ausmessen des dünnsten Querschnitts quantitativ bestimmt. Hierbei wurden in einem Beispielfall Werte von 39 µm ±16 µm gemessen.

Die Dicke der durch die erfindungsgemäßen Verfahren geschaffenen Oberflächenschicht liegt im Bereich von 0,1 mm bis 2,5 mm, vorzugsweise im Bereich von 0,3 mm bis 1,9 mm und besonders bevorzugt im Bereich von 0,5 mm bis 1,5 mm.

Der maximale Porendurchmesser liegt im Bereich von 10 µm bis 800 µm, bevorzugt im Bereich von 50 µm bis 600 µm und besonders bevorzugt im Bereich von 100 µm bis 400 µm.

Ein Porenmund der Poren, d. h. deren Öffnung nach außen, liegt im Bereich von 10 µm bis 900 µm, bevorzugt im Bereich von 75 µm bis 680 µm und besonders bevorzugt im Bereich von 150 µm bis 500 µm.

Eine Porosität der Oberflächenschicht liegt im Bereich von 20 % bis 85 %, vorzugsweise im Bereich von 30 % bis 70 % und besonders bevorzugt im Bereich von 40 % bis 50 %, wobei nochmals betont sei, daß sich das zusammenhängende Porennetzwerk bis zu der Rohoberfläche des Implantats und/oder einer optionalen Zwischenschicht erstreckt, die zwischen der Oberflächenschicht und einer Rohoberfläche des Implantats angeordnet sein kann.

Die Oberflächenschicht ist aus, vorzugsweise kantigen, Titanpartikeln gebildet, die mit Siliziumpartikeln beschichtet, insbesondere compoundiert sind. Die Menge der zur Compoundierung verwendeten Siliziumpartikel liegt im Bereich von 0,5 Gew.-% bis 8,5 Gew.-% ± 1,5 Gew.-%, vorzugsweise 0,5 Gew.-% bis 3,0 Gew.-% und besonders bevorzugt 1,0 Gew.-% ± 0,5 Gew.-%.

Als Titanpartikel wird bevorzugt ein Titanpulver verwendet, dessen Partikel eine Korngröße im Bereich von 90 µm bis < 200 µm, vorzugsweise im Bereich von 150 µm bis < 200 µm aufweisen. Als Siliziumpartikel wird bevorzugt Siliziumpulver einer Korngröße von ≤ 80 µm, vorzugsweise ≤ 40 µm und besonders bevorzugt von ≤ 20 µm, verwendet, so daß mit Siliziumpartikeln beschichtete Titanpartikel als Compoundpartikel vorliegen, die ein Achsenverhältnis von ≤ 5 : 2 aufweisen. Eine in Volumenprozent bezüglich der Korngröße gemäß ISO/DIS 13322-1 gemessene Korngrößenverteilung ist in nachfolgender Tabelle angegeben:

| Korngrößenverteilung [Vol.- %] | Korngröße [µm] |
|---|---|
| D10 | 90 - 149 |
| D50 | 15 - 229 |
| D90 | 230 - 330 |

Die mit Siliziumpartikeln beschichteten Titanpartikel, welche als Compoundpartikel vorliegen, weisen eine Korngröße von ≥ 90 µm, vorzugsweise ≥ 150 µm auf, wobei die oben definierte Korngrößenverteilung eingehalten wird.

Zwischen der Rohoberfläche des Implantats und der Oberflächenschicht ist vorzugsweise eine Zwischenschicht vorgesehen. Eine solche Zwischenschicht ist beispielsweise dann zweckmäßig, wenn sich der Grundstoff des zu beschichtenden Substrats nicht direkt für eine Beschichtung mittels Vakuum-Plasma-Spritzen eignet oder eine extrem hohe Festigkeit erforderlich ist, die sich beispielsweise nach der jeweiligen Anwendung richtet. In diesem Fall kann die Rohoberfläche des Implantats zunächst mit einer dichten Grundschicht überzogen werden, die vorzugsweise aus einem bioinerten Material besteht, das vorzugsweise ein Metall ist. Als Metall ist aufgrund seiner bioinerten Eigenschaften und seiner sehr hohen Festigkeit sowie seines geringen spezifischen Gewichts Titan bevorzugt. Es kommt jedoch auch Silizium oder mit Silizium compoundiertes Titan in Betracht. Andere bioinerte Metalle, wie beispielsweise Tantal, Platin oder andere Edelmetalle kommen ebenso als Grundschichtmaterialien in Betracht.

Die Zwischenschicht weist eine Schichtdicke von ≤ 200 µm, vorzugsweise von ≤ 100 µm und besonders bevorzugt im Bereich von 30 µm bis 50 µm auf.

Ferner sind an der Oberflächenschicht vorzugsweise bioaktive Partikel, insbesondere aus Titandioxid und/oder Kalziumphosphat, vorzugsweise Hydroxyapatit und/oder Trikalziumphosphat, angeordnet. Die bioaktiven Partikel weisen eine Korngröße im Bereich von 0,01 µm bis 5 µm, vorzugsweise im Bereich von 0,1 µm bis 3 µm und besonders bevorzugt im Bereich von 0,2 µm bis 1 µm auf, wobei in besonderen Fällen auch Partikel von ≤ 0,01 µm Anwendung finden können. Diese Pulver können mittels Sol-Gel-Verfahren mit einem Binder auf Wasser- oder Silikatbasis, mittels Ausfällen oder mittels Elektrophorese aufgebracht werden, wobei hinsichtlich der Applikationsart keine Einschränkungen bestehen und sämtliche gängigen und/oder geeigneten Applikationsverfahren zur Anwendung kommen können.

Mittels der durch das erfindungsgemäße Verfahren geschaffenen Oberflächenschicht ist somit eine Möglichkeit geschaffen, eine optimale Verankerung eines Knochen an bzw. in einem Implantat, respektive dessen Oberflächenschicht, zu gewährleisten, wobei ein Knocheneinwachsen und ein Knochenanwachsen erreicht wird. Durch die erfindungsgemäße Oberflächenschicht, die gegebenenfalls über eine stabilitäts- und festigkeitsvermittelnde Zwischenschicht in optimaler Weise mit einer Rohoberfläche des Implantats verbunden ist, ist es möglich, eine Knochenverankerung zur Verfügung zu stellen, die in einem einfachen und einstufigen Verfahren herstellbar ist, und bei welcher der Grundwerkstoff des Implantats thermisch nur sehr gering belastet wird.

Da die Oberflächenschicht aus einzelnen miteinander verbundenen Metallpartikeln aufgebaut ist, ist eine Variation der Art und Größe bzw. Dimensionierung der Metallpartikel an jeweils gewünschte Eigenschaften gut anpaßbar, da die Partikel schichtweise aufgetragen und über einen thermischen Prozeß miteinander verbunden werden. Auf dieses Weise ist es möglich, eine Oberfläche mit einer sehr guten Biokompatibilität im Sinne einer bioinerten oder leicht bioaktiven Eigenschaft herzustellen, die eine inhärent große innere Oberfläche sowie eine sehr gute mechanische Festigkeit und Stabilität aufweist und auch hinsichtlich Abrieb, selbst bei abrasiver Beanspruchung, nur eine äußerst geringe Abnutzung aufweist und deshalb die Lebensdauer und den Tragekomfort von Implantaten, insbesondere gegenüber dem derzeitigen Stand der Technik, maßgeblich erhöht und verbessert.

Weiterhin wird die Aufgabe durch ein Verfahren zur Herstellung einer offenporigen biokompatiblen Oberflächenschicht für ein Implantat, insbesondere Gelenkersatzimplantat, gelöst, wobei die folgenden Schritte durchgeführt werden:
- Beschichten von Titanpartikeln mit Silizium, das mit Titan oder einer Legierung daraus ein Eutektikum bildet, das bei einer niedrigeren Temperatur schmilzt als reines Titan, zur Erzeugung von Compoundpartikeln,
- Aufbringen wenigstens einer Schicht der Compoundpartikel mittels eines Vakuum-Plasma-Spritzverfahrens auf eine Rohoberfläche des Implantats zur Erzeugung einer Oberflächenschicht an dem Implantat, und
- Erzeugens eines Lichtbogens zwischen der Rohoberfläche des Implantats und einer Gegenelektrode während und/oder nach dem Aufbringen der Compoundpartikel.

Ferner wird die Aufgabe durch ein Verfahren zur Herstellung einer offenporigen biokompatiblen Schicht für ein Implantat, insbesondere Gelenkersatzimplantat gelöst, wobei die folgenden Schritte durchgeführt werden:
- Aufbringen wenigstens einer Schicht von Titanpartikeln mittels eines Vakuum-Plasma-Spritzverfahrens auf ein Rohoberfläche des Implantats zur Erzeugung einer Oberflächenschicht an dem Implantat,
- Erzeugen eines Lichtbogens zwischen der Rohoberfläche des Implantats und einer Gegenelektrode, und
- Erzeugens eines Lichtbogens zwischen der Rohoberfläche des Implantats und einer Gegenelektrode während und/oder nach dem Aufbringen der Titanpartikel.

Erfindungsgemäß kann der Lichtbogen zwischen der Rohoberfläche des Implantats, respektive einer an dem Implantat befindlichen Oberflächenschicht und einer Gegenelektrode unmittelbar während des Aufbringens der Titan- und/oder Compoundpartikel auf das Implantat erfolgen; alternativ kann der Lichtbogen jedoch auch nach einem Aufbringen der Titan- bzw. Compoundpartikel erfolgen. Eine weitere Alternative besteht in der Anwendung des Lichtbogenverfahrens während und nach der Aufbringung der Titan- und/oder Compoundpartikel.

Im ersteren Fall, nämlich im Falle des gleichzeitigen Aufbringens der Partikel und der Anwendung des Lichtbogens, kann zusätzlich zu dem internen, plasmabildenden Stromkreis des Beschichtungsverfahrens ein zweiter Stromkreis zwischen dem Plasmatron, respektive der Plasmapistole, und dem Bauteil geschaltet werden, so dass über das elektrisch leitfähige Plasma ein übertragener Lichtbogen eingestellt werden kann. Es ist zur Erzeugung des Lichtbogens auch möglich, diesen nicht ausgehend von dem Plasmatrom bzw. der Spritzpistole, sondern mittels einer bereitgestellten Gegenelektrode zu erzeugen.

Die Verwendung eines Lichtbogens während oder nach der Aufbringung von Titan- bzw. Compoundpartikeln auf einer Rohoberfläche eines Implantats weist den wesentlichen Vorteil einer Joul'schen Erwärmung an hochohmigen Übergangswiderständen, d.h. in diesem Fall an den Kontaktstellen zwischen den Titan- bzw. Compoundkörnern, respektive -partikeln auf, da diese verengte Stellen und damit einen erhöhten elektrischen Widerstand darstellen. Darüber hinaus stellt, insbesondere bei Compoundpartikeln die Überzugsschicht des Sinterhilfsmittels an den damit beschichteten Titanpartikeln einen höherohmigen Widerstand dar, als die Titanpartikel selbst, so dass auch hier Joul'sche Wärme entsteht, was zu einem lokalen nur auf die Berührungsstellen der Körner begrenzten Erwärmung, insbesondere in der Überzugsschicht, führt, durch welche die die Partikel verbindenden Sinterhälse verstärkt werden.

Zusammengefasst führt diese an Ort und Stelle frei gesetzte Wärme zu einer zusätzlichen lokalen Versinterung und dadurch zu einer Verstärkung der Sinterhälse zwischen den Titankörner, ohne jedoch gleichzeitig ein Versintern und/oder Einglätten der übrigen Bereiche der Partikel sowie der Substratoberfläche zu bewirken. Dieser vorteilhafte Effekt bewirkt in der Beschichtung eine starke Erhöhung von deren Scherfestigkeit um einen Faktor von 2 bis 3 gegenüber den gleichen Beschichtungsparametern bei der Herstellung, jedoch ohne übertragenen Lichtbogen. Gegenüber herkömmlichen Beschichtungsverfahren kann durch die gemeinsame Verwendung eines Sinterhilfsmittels und eines übertragenen Lichtbogens eine sechs- bis siebenfach erhöhte Scherfestigkeit gegenüber einer reinen offenporösen Titanschicht erreicht werden.

An dieser Stelle sei erwähnt, dass als Lichtbogen jede Art der Energieeinwirkung, insbesondere elektrischer Art zu verstehen ist, durch die ein Joul'scher Wärmeeffekt an verengten Stellen erzielt werden kann. Ein wesentlicher Vorteil der Verwendung eines Lichtbogens besteht jedoch darin, dass dieser unmittelbar während der Aufbringung der Titan- bzw. Compoundpartikel angewendet werden kann, so dass der Beschichtungsprozess letztlich einstufig abläuft und keinen zusätzlichen Verfahrensschritt erfordert, wenngleich auch eine nachträgliche Verstärkung der Sinterhälse durch die Anwendung eines Lichtbogens denkbar ist.

Im übrigen sei darauf hingewiesen, dass der Übergangswiderstand, welcher durch das Sinterhilfsmittel bedingt ist, durch die Wahl des Sinterhilfsmittels und durch dessen Schichtdicke erfindungsgemäß beeinflusst werden kann, so dass eine gezielte sehr hohe Verfestigung der offenporigen Oberflächenschicht mit einem zusammenhängenden Porennetzwerk erreicht werden kann.

Erfindungsgemäß werden als Sinterhilfsmittel Silizium oder Kobalt verwendet, wobei im Falle der Verwendung von Silizium vorzugsweise Siliziumpartikel in einer Menge im Bereich von 0,5 Gew.-% bis 8,5 Gew.-% ± 1,5 Gew.-%, vorzugsweise 0,5 Gew.-% bis 3,0 Gew.-% und besonders bevorzugt 1,0 Gew.-% ± 0,5 Gew.-%, verwendet werden.

Ferner wird als Titanpartikel ein kantiges Titanpulver mit einer Korngröße im Bereich von 90 µm bis < 200 µm, vorzugsweise im Bereich von 150 bis < 200 µm verwendet, das vorzugsweise gemahlen und erfindungsgemäß vorzugsweise über die Hydridstufe hergestellt wurde.

Als Siliziumpartikel wird Siliziumpulver mit einer Korngröße ≤ 80 µm, vorzugsweise ≤ 40 µm und besonders bevorzugt von ≤ 20 µm verwendet. Bezüglich der Korngrößenverteilung wird auf vorstehende Ausführungen verwiesen.

Die Titanpartikel werden so mit Siliziumpartikeln beschichtet, daß die nunmehr als Compoundpartikel bezeichneten beschichteten Titanpartikel eine Korngröße ≥ 90 µm, vorzugsweise ≤ 150 µm aufweisen, wobei wiederum die vordefinierte Korngrößenverteilung zu beachten ist.

Aufgrund dieser Größenverhältnisse ist es möglich, die Titanpartikel mit Siliziumpartikeln zu beschichten und auf diese Weise eine hohe Oberfläche zur Verfügung zu stellen, die bei einem späteren Aufschmelzen während eines Vakuum-Plasma-Beschichtungsprozesses zum Verbinden von Partikeln, respektive zum Verbinden von Partikeln mit einem Substrat zur Verfügung steht.

Ferner ist es unter Einhaltung der vorgenannten Korngrößen möglich, Titanpartikel so mit Siliziumpartikeln zu beschichten, daß eine kantige Peripherie der beschichteten Titanpartikel, d. h. der Compoundpartikel, im wesentlichen erhalten bleibt und die Compoundpartikel ein Achsenverhältnis von ≤ 5 : 2 aufweisen.

Des weiteren werden die Vakuum-Plasma-Spritzverfahrensparameter so eingestellt, daß die Compoundpartikel nur oberflächlich angeschmolzen und beim Auftreffen auf die Rohoberfläche des Implantats, respektive auf die Zwischenschicht, sofern vorhanden, nur geringfügig kompaktiert werden, so daß deren ursprüngliche Geometrie, insbesondere hinsichtlich deren Achsenverhältnis, weitgehend erhalten bleibt. Auf diese Weise ist es möglich, mittels der kantigen Partikel, die auf einer Substratoberfläche auftreffen, ein Gefüge zu bilden, das im wesentlichen nur über Punkte und Kanten der einzelnen kantigen Partikel miteinander verbunden ist, so daß eine Art Käfig entsteht, aus dem das zusammenhängende Porennetzwerk gebildet wird und durch dessen Hohlräume Knochensubstanz an- und einwachsen kann.

Wie vorerwähnt, ist es möglich, daß die Rohoberfläche des Implantats vor einem Beschichtungsvorgang mit Compoundpartikeln mit einer Grundschicht, d. h. mit einer Zwischenschicht, beschichtet wird, die je nach Basismaterial eines Implantat-Rohkörpers ausgebildet sein kann und vorzugsweise aus reinem Titan und/oder aus mit Silizium beschichtetem Titan besteht.

Gemäß einer bevorzugten Ausführungsform erfolgt das Aufbringen der Zwischenschicht im selben Vakuum-Plasma-Spritzbeschichtungsvorgang wie das Aufbringen der eigentlichen Oberflächenschicht, wobei selbsterklärend das Aufbringen der Zwischenschicht vor dem Aufbringen der Oberflächenschicht erfolgt.

Die Grund- bzw. Zwischenschicht wird mit einer Schichtdicke von ≤ 200 µm, vorzugsweise von ≤ 100 µm und besonders bevorzugt im Bereich von 30 µm bis 50 µm hergestellt.

Im übrigen ist erfindungsgemäß vorgesehen, daß auf die Oberflächenschicht bioaktive Partikel, insbesondere aus Titandioxid und/oder Kalziumphosphat, vorzugsweise Hydroxyapatit und/oder Trikalziumphosphat, mit einer Korngröße im Bereich von 0,01 µm bis 5 µm, vorzugsweise im Bereich von 0,1 µm bis 3 µm und besonders bevorzugt im Bereich von 0,2 µm bis 1 µm aufgebracht werden.

Durch die Aufbringung solcher Partikel kann eine Beschleunigung des Anwachsens und des Einwachsens von Knochen in die offenporigen Kanäle der Oberflächenschicht beschleunigt und verbessert werden. Diese bioaktiven Partikel werden vorzugsweise, wie vorerwähnt, als Pulver eingesetzt und können im Sol-Gel-Verfahren mit einem Binder auf Wasser- oder Silikatbasis, mittels Ausfällen oder Elektrophorese aufgebracht werden.

Des weiteren liegt es im Umfang der Erfindung, daß wenigstens eine über der Rohoberfläche des Implantats aufgebrachte Schicht, vorzugsweise im Vakuum, gesintert wird. Hierdurch kann, sofern gewünscht, eine Verbindung zwischen den einzelnen Partikeln bzw. zwischen einzelnen Partikeln und dem Substrat durch eine Verstärkung des Sinterhalses verbessert werden. Es sei jedoch erwähnt, daß ein solches Sintern allenfalls sehr kurzzeitig erfolgt, um ein Einglätten der Mikrostruktur der Oberflächenbeschichtung zu vermeiden. In aller Regel ist ein solches Kurzzeit-Sintern aufgrund des Einsatzes der erfindungsgemäßen Compoundpartikel nicht notwendig, sondern kann in Einzelfällen zur Verstärkung der Sinterhälse angewandt werden.

Erfindungsgemäß wird eine Schichtdicke der Oberflächenschicht im Bereich von 0,1 mm bis 2,5 mm, vorzugsweise im Bereich von 0,3 mm bis 1,9 mm und besonders bevorzugt im Bereich von 0,5 mm bis 1,5 mm erzeugt.

Eine durch die erfindungsgemäßen Verfahren geschaffene Oberflächenschicht gemäß der vorstehenden Spezifikation bzw. dem vorstehend genannten Herstellverfahren kann beispielsweise für Hüftschäfte, Schalen für Hüftgelenke, Femurkomponenten für einen Kniegelenkersatz, Tibiakomponenten für einen Kniegelenkersatz, Komponenten für einen Schultergelenkersatz, Komponenten für einen Ellbogengelenkersatz, Komponenten für einen Zehengelenkersatz, Komponenten für einen Fingergelenkersatz, für eine Komponente zur Fusion von Wirbelkörpern der Lumbarwirbelsäule, für Komponenten für einen Bandscheibenersatz, für transgingivale Implantatsysteme, für orthodontische Implantatsysteme und Zahn(ersatz)-implantate eingesetzt werden.

Wie bereits vorerwähnt, eignet sich die durch die erfindungsgemäßen Verfahren geschaffene Oberflächenschicht für die vorstehend genannten Anwendungsgebiete in hervorragender Weise, da eine zementfreie Verankerung von Knochen an den Implantaten aufgrund der hervorragenden Eigenschaften der erfindungsgemäßen Oberflächenschicht gewährleistet ist, die ein optimiertes Ein- und Anwachsen von Knochen an der Oberflächenschicht und damit an dem Implantat gewährleisten.

Weitere Ausführungsformen der Erfindung ergeben sich aus den Unteransprüchen.

Nachfolgend wird die Erfindung anhand eines Ausführungsbeispiels beschrieben, das anhand der Abbildungen näher erläutert wird. Hierbei zeigen:
- Fig. 1: ein auf einer mittels Korundstrahlen aufgerauhten Implantatoberfläche einer Prothese aus Ti6A17Nb-Legierung angewachsenes Knochenbälkchen gemäß dem Stand der Technik;
- Fig. 2: eine "Beads"-Beschichtung aus einer CoCr-Legierung mit eingewachsenem Knochen als Makroaufnahme gemäß dem Stand der Technik;
- Fig. 3: eine rasterelektronenmikroskopische Aufnahme in 50-facher Vergrößerung einer über einen Sinterprozeß hergestellten Titanbeschichtung gemäß dem Stand der Technik;
- Fig. 4: die Titanbeschichtung aus Fig. 3 in 1000-flacher Vergrößerung;
- Fig. 5: einen metallographischen Schliff durch eine Vakuum-Plasma-gespritzte Titanschicht in 100-facher Vergrößerung mit einer guten Darstellung einer hohen Rauheit, jedoch wenig vorhandenen Hohlräümen;
- Fig. 6: einen metallographischen Schliff durch eine Vakuum-Plasma-gespritzte erfindungsgemäße Titanschicht in 20-facher Vergrößerung;
- Fig. 7: eine rasterelektronenmikroskopische Aufnahme der Oberfläche eines Titanpartikels in der erfindungsgemäßen Vakuum-Plasma-gespritzten Titanschicht in 1000-facher Vergrößerung; und
- Fig. 8: eine schematische Darstellung des erfindungsgemäßen Lichtbogenverfahrens.

Ausgehend von einem gemahlenen, kantigen, über die Hydridstufe hergestellten Titanpulver wird dieses in einem ersten Schritt mit einer geringen Menge eines feinen Siliziumpulvers compoundiert. Das Titanpulver weist dabei eine Korngröße von kleiner 200 µm auf und das Siliziumpulver eine solche von maximal 65 µm. Das so entstandene Spritzpulver bzw. die so entstandenen Compoundpartikel bestehen aus nach wie vor kantigen Partikeln mit einem Achsenverhältnis von maximal 5:2. Als nächstes wird dieses compoundierte Pulver als offenporige Struktur auf die Implantatoberfläche aufgebracht. Die offenporige Schicht selbst hat eine Schichtdicke von 0,5 mm bis 1,5 mm und eine Porosität von 40 % bis 50 %. Die Größe der Poren, bestimmt als maximaler Durchmesser, beträgt 100 µm bis 400 µm. Die Öffnung dieser Poren gegen außen liegt im Bereich von 150 µm bis 500 µm. Diese Schicht wird mittels eines Vakuum-Plasma-Spritzverfahrens sowie unter Verwendung eines simultanen Lichtbogens aufgebracht. Die Plasma-Spritzparameter werden bei diesem Verfahren so eingestellt, daß die Titanpartikel zwar leicht angeschmolzen werden, aber beim Aufprall auf das Substrat, nämlich die Rohoberfläche des Implantats, nur geringfügig kompaktiert werden. Durch den angewendeten Lichtbogen werden die aufgebrachten Titanpartikel zusätzlich miteinander verbunden sowie die sie verbindenden Sinterhälse und deren Verbindung mit dem Substrat, d.h. der Oberfläche des Implantats, verstärkt. Es entsteht ein zusammenhängendes Netzwerk von gegenseitig verbundenen Titanpartikeln und gleichzeitig eine zusammenhängende, innere Porosität. Diese ist als metallographischer Schliff in 20-facher Vergrößerung in Fig. 6 dargestellt, wobei das Metall hell und die Hohlräume dunkel erscheinen.

Die Porosität der erfindungsgemäßen mittels eines Vakuum-Plasma-Spritzverfahrens aufgebrachten Titanschicht ist bis zu dem Substrat durchgängig.

In nachfolgender tabellarischer Übersicht sind die geometrischen Meßwerte, durch welche diese erfindungsgemäße Schicht charakterisiert ist, zusammengefaßt:

| | Schicht dicke [µm] | Porosität [%] | Porengröße [µm] | Porenöffnung [µm] | Sinterhals [µm] | Innere Oberfläche [µm/µm²] |
|---|---|---|---|---|---|---|
| Ti-VPS, Erfindungsgemäß | 1120 | 46.0 ± 1.7 | 185 ± 60 | 312 ± 162 | 39 ± 16 | 0.0289 |

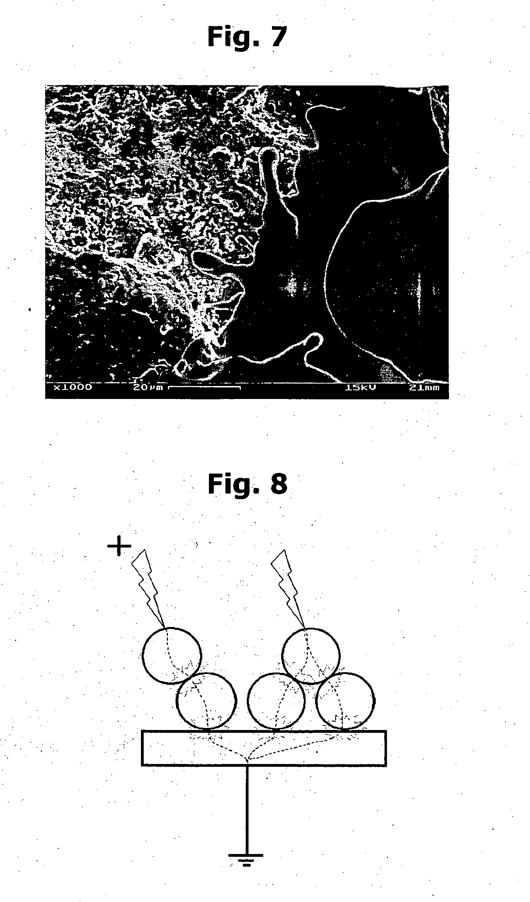

In Fig. 7, die eine rasterelektronenmikroskopische Aufnahme der Oberfläche eines erfindungsgemäß aufgebrachten Titanpartikels mittels Vakuum-Plasma-Spritzen in einer erfindungsgemäßen Titanschicht darstellt, ist gut zu erkennen, daß die mit Silizium compoundierten Titanpartikel, die zumindest in Teilbereichen ein Eutektikum bilden, während des Spritzprozesses nur oberflächlich und auch dort nur teilweise aufschmelzen. Sie behalten ihre ursprüngliche Geometrie mit Achslängen ähnlicher Größe. Auf der anderen Seite, nämlich der nicht aufgeschmolzenen, bleibt die Rauheit der compoundierten Titanpartikel erhalten. Verglichen mit sintertechnisch hergestellten Schichten, beispielsweise gemäß dem US-Patent 4,206,516, hat die erfindungsgemäße Schicht eine markant höhere spezifische Oberfläche. Ein Maß für die spezifische innere Oberfläche ist die im metallographischen Schliff bei 100-facher Vergrößerung auf den Bildausschnitt normierte, gemessene, innere Berandungslinie. Die in der Tabelle angegebene Schichtdicke wurde nach ASTM F 1854 bestimmt. Die anderen Meßwerte wurden bildanalytisch bestimmt.

Fig. 8 zeigt eine schematische Darstellung des angewandten Lichtbogenverfahrens, wobei das Substrat, auf welchem die kugelförmig dargestellten Teilchen aufgebracht werden sollen, als Elektrode bzw. als Erdung dient. Durch den Lichtbogen, der in die aufgebrachten Partikel einkoppelt, wird an deren Verbindungsstellen untereinander und mit dem Substrat, die einen höherohmigen Übergangswiderstand darstellen, eine Joul'sche Erwärmung erzeugt, die bewirkt, daß eine lokale Versinterung von diesen Verbindungsstellen erfolgt, ohne dass die Porosität und Rauhigkeit in übrigen Bereichen sowohl des Substrats als auch der aufgebrachten Partikel beeinflusst wird. Somit bleibt die Rauhigkeit und durchgängige Porosität, welche für ein Einwachsen von Knochengewebe optimal geeignet ist, nachhaltig erhalten, während die Festigkeit und der Zusammenhalt der Partikel untereinander und mit dem Substrat aufgrund der Anwendung des Lichtbogenverfahrens nachhaltig verbessert wird.

Die Vakuum-Plasma-aufgespritzten Schichten, insbesondere in Kombination mit dem Lichtbogenverfahren, weisen eine gute Haftfestigkeit gegenüber dem Substrat sowie hinsichtlich der Partikel untereinander und eine gute Scherfestigkeit innerhalb der Schicht auf. Im Haftzugversuch erreichte die Schicht Werte von 22,1 ± 4,1 MPa.

Die Haftfestigkeit einzelner Partikel wurde in einem modifizierten Miller-Test bestimmt. Dieses Testverfahren ist in ASTM G 75-01 beschrieben. In Abänderung zur ASTM-Norm wurde auf eine abrasive Suspension verzichtet und die beschichteten Proben unter einer Last von 22,4 N direkt und ohne weiteren Hilfsstoff über die Elastomer-Unterlage (Neopren) gezogen. Der so erzielte Abrieb einzelner Partikel der erfindungsgemäßen Oberflächen, normiert auf die Porosität der Schicht, ist kleiner und somit besser als derjenige einer Titanschicht gemäß dem Stand der Technik. Folglich ist auch der Zusammenhalt der einzelnen Partikel in der erfindungsgemäßen Oberflächenschicht besser. Dies ist dadurch begründet, daß die als Sinterhilfsmittel auf die Titanpartikel aufgebrachten Siliziumpartikel ein gezieltes Aufschmelzen der Oberfläche fördern und so über ein kurzzeitiges Flüssigphasen-Sintern eine stabile Verbindung der Partikel sowohl gegenüber dem Substrat als auch untereinander ermöglichen.

Zusammenfassend läßt sich festhalten, daß das erfindungsgemäße Verfahren zum Herstellen einer offenporigen biokompatiblen Oberflächenschicht für ein Implantat einen effektiven einstufigen Prozeß darstellt, mittels dem es möglich ist, ein zusammenhängendes Porennetzwerk mit hoher innerer Oberfläche, guter Scherfestigkeit, einer optimierten Poren- und Schichtdickengröße und hoher Biokompatibilität herzustellen, durch das ein effektives und schnelles Ein- und Anwachsen von Knochen an ein Implantat ermöglicht wird und das gegenüber dem bisherigen Stand der Technik hinsichtlich seiner Fähigkeit, ein Ein- und Anwachsen von Knochen zu ermöglichen, deutlich verbessert ist, wobei die Dauerhaftigkeit der erfindungsgemäßen Oberflächenschicht darüber hinaus deutlich verlängert ist, so daß ein implantierte Gelenkersatz deutlich länger im Körper verbleiben kann als bislang.

An dieser Stelle sei darauf hingewiesen, daß alle oben beschriebenen Teile für sich alleine gesehen und in jeder Kombination, insbesondere die in den Zeichnungen dargestellten Details, als erfindungswesentlich beansprucht werden. Abänderungen hiervon sind dem Fachmann geläufig.

## Patentansprüche

1. Verfahren zur Herstellung einer offenporigen biokompatiblen Oberflächenschicht für ein Implantat, insbesondere Gelenkersatzimplantat,
**gekennzeichnet durch**
die folgenden Schritte:
- Beschichten von Titanpartikeln mit einem Sinterhilfsmittel, das mit Titan oder einer Legierung daraus ein Eutektikum bildet, das bei einer niedrigeren Temperatur schmilzt als reines Titan, zur Erzeugung von Compoundpartikeln,
- Aufbringen wenigstens einer Schicht der Compoundpartikel mittels eines Vakuum-Plasma-Spritzverfahrens auf eine Rohoberfläche des Implantats zur Erzeugung einer Oberflächenschicht an dem Implantat, und
- Erzeugens eines Lichtbogens zwischen der Rohoberfläche des Implantats und einer Gegenelektrode während und/oder nach dem Aufbringen der Compoundpartikel.

2. Verfahren zur Herstellung einer offenporigen biokompatiblen Oberflächenschicht für ein Implantat, insbesondere Gelenkersatzimplantat,
**gekennzeichnet durch**
die folgenden Schritte:
- Aufbringen wenigstens einer Schicht von Titanpartikeln mittels eines Vakuum-Plasma-Spritzverfahrens auf eine Rohoberfläche des Implantats zur Erzeugung einer Oberflächenschicht an dem Implantat,
- Erzeugen eines Lichtbogens zwischen der Rohoberfläche des Implantats und einer Gegenelektrode, und
- Erzeugens eines Lichtbogens zwischen der Rohoberfläche des Implantats und einer Gegenelektrode während und/oder nach dem Aufbringen der Titanpartikel.

3. Verfahren nach einem der Ansprüche 1 oder 2,
**dadurch gekennzeichnet, daß**
als Sinterhilfsmittel Silizium oder Kobalt, vorzugsweise Siliziumpartikeln in einer Menge im Bereich von 0,5 Gew.-% bis 8,5 Gew.-% ± 1,5 Gew.-%, vorzugsweise 0,5 Gew.-% bis 3,0 Gew.-% und besonders bevorzugt 1,0 Gew.-% ± 0,5 Gew.-%, verwendet werden.

4. Verfahren nach einem der Ansprüche 1 bis 3,
**dadurch gekennzeichnet, daß**
als Titanpartikel ein kantiges Ti-Pulver mit einer Korngröße im Bereich von 90 µm bis < 200, vorzugsweise im Bereich von 150 µm bis < 200 µm verwendet wird.

5. Verfahren nach einem der Ansprüche 3 oder 4,
**dadurch gekennzeichnet, daß**
als Siliziumpartikel Siliziumpulver mit einer Korngröße ≤ 80 µm, vorzugsweise ≤ 40 µm und besonders bevorzugt ≤ 20 µm verwendet wird, wobei eine Korngrössenverteilung nach ISO/DIS 13322-1 (in Volumenprozent bezüglich der Korngrösse) gemäß D10: 90 µm bis 149 µm, gemäß D50: 150 µm bis 229 µm und gemäß D90: 230 µm bis 330 µm, definiert ist.

6. Verfahren nach einem der Ansprüche 1 bis 5,
**dadurch gekennzeichnet, daß**
die Titanpartikel mit Siliziumpartikeln beschichtet werden, derart, daß die Compoundpartikel eine Korngröße ≥ 90 µm, vorzugsweise ≥ 150 µm aufweisen, wobei eine Korngrössenverteilung nach ISO/DIS 13322-1 (in Volumenprozent bezüglich der Korngrösse) gemäß D10: 90 µm bis 149 µm, gemäß D50: 150 µm bis 229 µm und gemäß D90: 230 µm bis 330 µm, definiert ist.

7. Verfahren nach einem der Ansprüche 1 bis 3,
**dadurch gekennzeichnet, daß**
die Titanpartikel so mit Siliziumpartikeln beschichtet werden, dass eine kantige Peripherie der beschichteten Titanpartikel im wesentlichen erhalten bleibt und die mit Siliziumpartikeln beschichteten Titanpartikel ein Achsenverhältnis von ≤ 5: 2 aufweisen.

8. Verfahren nach einem der Ansprüche 1 bis 7,
**dadurch gekennzeichnet, daß**
die Vakuum-Plasma-Spritzverfahrensparameter so eingestellt werden, dass die Compoundpartikel und/oder Titanpartikel nur oberflächlich angeschmolzen und beim Auftreffen auf die Rohoberfläche des Implantats nur geringfügig kompaktiert werden, so dass deren ursprüngliche Geometrie, insbesondere hinsichtlich deren Achsenverhältnis, weitgehend erhalten bleibt.

9. Verfahren nach einem der Ansprüche 1 bis 8,
**dadurch gekennzeichnet, daß**
die Rohoberfläche des Implantats vor einem Beschichtungsvorgang mit Compoundpartikeln mit einer Grundschicht beschichtet wird.

10. Verfahren nach Anspruch 9,
**dadurch gekennzeichnet, daß**
als Material für die Grundschicht Pulver aus reinem Titan und/oder aus mit Silizium beschichteten Titan verwendet wird.

11. Verfahren nach einem der Ansprüche 9 oder 10,
**dadurch gekennzeichnet, daß**
das Beschichten der Rohoberfläche des Implantats mit der Grundschicht im selben Vakuum-Plasma-Spritzverfahrensschritt wie das Aufbringen der Compoundpartikel erfolgt.

12. Verfahren nach einem der Ansprüche 9 bis 11,
**dadurch gekennzeichnet, daß**
die Grundschicht mit einer Schichtdicke von ≤ 200 µm, vorzugsweise von ≤ 100 µm und besonders bevorzugt im Bereich von 30 µm bis 50 µm hergestellt wird.

13. Verfahren nach einem der Ansprüche 1 bis 12,
**dadurch gekennzeichnet, daß**
auf die Oberflächenschicht bioaktive Partikel, insbesondere aus Titandioxid und/oder Kalziumphosphat, vorzugsweise Hydroxyapatit und/oder Trikalziumphosphat, mit einer Korngröße im Bereich von 0,01 µm bis 5 µm, vorzugsweise im Bereich von 0,1 µm bis 3 µm und besonders bevorzugt im Bereich von 0,2 µm bis 1 µm aufgebracht werden.

14. Verfahren nach Anspruch 13,
**dadurch gekennzeichnet, daß**
die bioaktiven Partikel mittels Sol-Gel-Verfahren, Ausfällen und/oder mittels Elektrophorese sowie gegebenenfalls unter Zuhilfenahme von Binder auf Wasser- und/oder Silikatbasis aufgebracht werden.

15. Verfahren nach einem der Ansprüche 1 bis 14,
**dadurch gekennzeichnet, daß**
die wenigstens eine über der Rohoberfläche des Implantats aufgebrachte Schicht, vorzugsweise im Vakuum, gesintert wird.

16. Verfahren nach einem der Ansprüche 1 bis 15,
**dadurch gekennzeichnet, daß**
eine Schichtdicke der Oberflächenschicht im Bereich von 0,1 mm bis 2,5 mm, vorzugsweise im Bereich von 0,3 mm bis 1,9 mm und besonders bevorzugt im Bereich von 0,5 mm bis 1,5 mm erzeugt wird.

## Claims

1. Method of producing an open-pore biocompatible surface layer for an implant, especially a joint replacement implant,
**characterised by**
the following steps:
- coating of titanium particles with a sintering adjuvant that forms with titanium or an alloy thereof a eutectic that melts at a lower temperature than pure titanium, to produce compound particles,
- application of at least one layer of the compound particles to a virgin surface of the implant by means of a vacuum plasma spraying method, to produce a surface layer on the implant, and
- production of an arc between the virgin surface of the implant and a counter-electrode during and/or after application of the compound particles.

2. Method of producing an open-pore biocompatible surface layer for an implant, especially a joint replacement implant,
**characterised by**
the following steps:
- application of at least one layer of titanium particles to a virgin surface of the implant by means of a vacuum plasma spraying method, to produce a surface layer on the implant,
- production of an arc between the virgin surface of the implant and a counter-electrode, and
- production of an arc between the virgin surface of the implant and a counter-electrode during and/or after application of the titanium particles.

3. Method according to either one of claims 1 and 2,
**characterised in that**
silicon or cobalt are used as sintering adjuvant, preferably silicon particles in a quantity in the range from 0.5 % by weight to 8.5 % by weight ± 1.5 % by weight, preferably from 0.5 % by weight to 3.0 % by weight and especially 1.0 % by weight ± 0.5 % by weight.

4. Method according to any one of claims 1 to 3,
**characterised in that**
an angular Ti powder having a particle size in the range from 90 µm to < 200 µm, preferably in the range from 150 µm to < 200 µm, is used as titanium particles.

5. Method according to either one of claims 3 and 4,
**characterised in that**
silicon powder having a particle size ≤ 80 µm, preferably ≤ 40 µm and especially ≤ 20 µm is used as silicon particles, wherein the particle size distribution is, in accordance with ISO/DIS 13322-1 (in percentage by volume with respect to the particle size), defined as corresponding to D10: from 90 µm to 149 µm, as corresponding to D50: from 150 µm to 229 µm, and as corresponding to D90: from 230 µm to 330 µm.

6. Method according to any one of claims 1 to 5,
**characterised in that**
the titanium particles are coated with silicon particles in such a manner that the compound particles have a particle size ≥ 90 µm, preferably ≥ 150 µm, wherein the particle size distribution is, in accordance with ISO/DIS 13322-1 (in percentage by volume with respect to the particle size), defined as corresponding to D10: from 90 µm to 149 µm, as corresponding to D50: from 150 µm to 229 µm, and as corresponding to D90: from 230 µm to 330 µm.

7. Method according to any one of claims 1 to 3,
**characterised in that**
the titanium particles are coated with silicon particles in such a manner that an angular periphery of the coated titanium particles is substantially preserved and the titanium particles coated with silicon particles have an axial ratio of ≤ 5:2.

8. Method according to any one of claims 1 to 7,
**characterised in that**
the parameters of the vacuum plasma spraying method are adjusted in such a manner that the compound particles and/or titanium particles are caused to start
to melt only superficially and, on meeting the virgin surface of the implant, are only slightly compacted, so that their original geometry, especially as regards their axial ratio, is to a great extent preserved.

9. Method according to any one of claims 1 to 8,
**characterised in that**
the virgin surface of the implant is coated with a base layer prior to a coating operation with compound particles.

10. Method according to claim 9,
**characterised in that**
powder composed of pure titanium and/or of titanium coated with silicon is used as the material for the base layer.

11. Method according to either one of claims 9 and 10,
**characterised in that**
the coating of the virgin surface of the implant with the base layer takes place in the same step of the vacuum plasma spraying method as the application of the compound particles.

12. Method according to any one of claims 9 to 11,
**characterised in that**
the base layer is produced with a layer thickness of ≤ 200 µm, preferably ≤ 100 µm and especially in the range from 30 µm to 50 µm.

13. Method according to any one of claims 1 to 12,
**characterised in that**
bioactive particles, especially of titanium dioxide and/or calcium phosphate, preferably hydroxyapatite and/or tricalcium phosphate, having a particle size in the range from 0.01 µm to 5 µm, preferably in the range from 0.1 µm to 3 µm and especially in the range from 0.2 µm to 1 µm, are applied to the surface layer.

14. Method according to claim 13,
**characterised in that**
the bioactive particles are applied by means of a sol-gel process, precipitation and/or by means of electrophoresis, and optionally with the assistance of water-based and/or silicate-based binder.

15. Method according to any one of claims 1 to 14,
**characterised in that**
the at least one layer applied over the virgin surface of the implant is sintered, preferably *in vacuo*.

16. Method according to any one of claims 1 to 15,
**characterised in that**
a layer thickness of the surface layer in the range from 0.1 mm to 2.5 mm, preferably in the range from 0.3 mm to 1.9 mm and especially in the range from 0.5 mm to 1.5 mm is produced.

## Revendications

1. Procédé de fabrication d'une couche superficielle biocompatible à pores ouverts pour un implant, en particulier un implant pour prothèse articulaire, **caractérisé par** les étapes suivantes consistant à :
- recouvrir les particules de titane d'un adjuvant de frittage, qui forme avec le titane ou un alliage de ce dernier un eutectique, qui fond à une température plus basse que le titane pur, pour la production de particules composites,
- appliquer au moins une couche de particules composites, par un procédé de projection de plasma sous vide, sur une surface brute de l'implant, pour produire une couche superficielle sur l'implant, et
- produire un arc électrique entre la surface brute de l'implant et une contre-électrode pendant et/ou après l'application des particules composites.

2. Procédé de fabrication d'une couche superficielle biocompatible à pores ouverts pour un implant, en particulier un implant pour prothèse articulaire, **caractérisé par** les étapes suivantes consistant à :
- appliquer au moins une couche de particules de titane, par un procédé de projection de plasma sous vide, sur une surface brute de l'implant pour la production d'une couche superficielle sur l'implant,
- produire un arc électrique entre la surface brute de l'implant et une contre-électrode, et
- produire un arc électrique entre la surface brute de l'implant et une contre-électrode pendant et/ou après l'application des particules de titane.

3. Procédé selon l'une quelconque des revendications 1 ou 2, **caractérisé en ce qu'**on utilise en tant qu'adjuvant de frittage du silicium ou du cobalt, de préférence des particules de silicium en une quantité située dans la plage allant de 0,5 % en poids à 8,5 % en poids ± 1,5 % en poids, de préférence de 0,5 % en poids à 3,0 % en poids et de manière particulièrement préférée de 1,0 % en poids ± 0,5 % en poids.

4. Procédé selon l'une quelconque des revendications 1 à 3, **caractérisé en ce qu'**on utilise en tant que particules de titane une poudre de Ti à arêtes vives, présentant une granulométrie située dans la plage allant de 90 µm à < 200, de préférence dans la plage de 150 µm à < 200 µm.

5. Procédé selon l'une quelconque des revendications 3 ou 4, **caractérisé en ce qu'**on utilise en tant que particules de silicium une poudre de silicium présentant une granulométrie ≤ 80 µm, de préférence ≤ 40 µm et d'une manière particulièrement préférée ≤ 20 µm, une distribution granulométrique selon l'ISO/DIS 13322-1 (en pourcent en volume concernant la granulométrie) étant définie selon D10 : 90 µm à 149 µm, selon D50 : 150 µm à 229 µm et selon D90 : 230 µm à 330 µm.

6. Procédé selon l'une quelconque des revendications 1 à 5, **caractérisé en ce que** les particules de titane sont recouvertes de particules de silicium de telle sorte que les particules composites présentent une granulométrie ≥ 90 µm, de préférence ≥ 150 µm, une distribution granulométrique selon ISO/DIS 13322-1 (en pourcent en volume concernant la granulométrie) étant définie selon D10 : 90 µm à 149 µm, selon D50 : 150 µm à 229 µm, et selon D90 : 230 µm à 330 µm.

7. Procédé selon l'une quelconque des revendications 1 à 3, **caractérisé en ce que** les particules de titane sont recouvertes de particules de silicium de telle sorte qu'une périphérie à arêtes vives des particules de titane recouvertes reste sensiblement, et que les particules de titane recouvertes de particules de silicium présentent un rapport d'axes ≤ 5:2.

8. Procédé selon l'une quelconque des revendications 1 à 7, **caractérisé en ce que** les paramètres du procédé de projection de plasma sous vide sont réglés de telle sorte que les particules composites et/ou les particules de titane ne fondent qu'en surface, et, lors de leur impact sur la surface brute de l'implant, ne sont que faiblement compactées, de sorte que leur géométrie initiale reste sensiblement, en particulier en ce qui concerne leur rapport d'axes.

9. Procédé selon l'une quelconque des revendications 1 à 8, **caractérisé en ce que** la surface brute de l'implant est recouverte, avant une opération de revêtement, de particules composites présentant une couche de base.

10. Procédé selon la revendication 9, **caractérisé en ce qu'**on utilise en tant que matériau pour la couche de base une poudre de titane pur et/ou de titane recouverte de silicium.

11. Procédé selon l'une quelconque des revendications 9 ou 10, **caractérisé en ce que** le revêtement de la surface brute de l'implant par la couche de base a lieu lors de la même étape du procédé de projection de plasma sous vide que l'application des particules composites.

12. Procédé selon l'une quelconque des revendications 9 à 11, **caractérisé en ce que** la couche de base est fabriquée avec une épaisseur de couche ≤ 200 µm, de préférence ≤ 100 µm et de manière particulièrement préférée située dans la plage allant de 30 µm à 50 µm.

13. Procédé selon l'une quelconque des revendications 1 à 12, **caractérisé en ce qu'**on applique sur la couche superficielle des particules bioactives, en particulier de dioxyde de titane et/ou de phosphate de calcium, de préférence d'hydroxyapatite et/ou de phosphate tricalcique, présentant une granulométrie située dans la plage allant de 0,01 µm à 5 µm, de préférence dans la plage de 0,1 µm à 3 µm et de manière particulièrement préférée dans la plage de 0,2 µm à 1 µm.

14. Procédé selon la revendication 13, **caractérisé en ce que** les particules bioactives sont appliquées par un procédé sol-gel, par précipitation et/ou par électrophorèse, ainsi que le cas échéant à l'aide d'un liant à base d'eau et/ou d'un silicate.

15. Procédé selon l'une quelconque des revendications 1 à 14, **caractérisé en ce que** l'au moins une couche appliquée sur la surface brute de l'implant est frittée de préférence sous vide.

16. Procédé selon l'une quelconque des revendications 1 à 15, **caractérisé en ce qu'**on produit une épaisseur de couche de la couche superficielle située dans la plage allant de 0,1 mm à 2,5 mm, de préférence dans la plage de 0,3 mm à 1,9 mm et de manière particulièrement préférée dans la plage allant de 0,5 mm à 1,5 mm.
